# EUROPEAN PATENT APPLICATION

(11) **EP 4 712 090 A1**
(43) Date of publication of application: **18.03.2026**
(21) Application number: 24848623.5
(22) Date of filing: 02.05.2024
(51) Int. Cl.: G16H 10/00

(54) **MEDICAL DEVICE SYSTEM, MEDICAL INFORMATION ACQUISITION METHOD, MEDICAL DEVICE, INFORMATION DEVICE, AND PROGRAM**

(30) Priority: 31.07.2023 JP 2023124372
(71) Applicant: Terumo Kabushiki Kaisha, Tokyo 151-0072 (JP)
(72) Inventor: YAKUSHIJI, Yusuke, Ashigarakami-gun, Kanagawa 259-0151 (JP)
(74) Representative: Prüfer & Partner mbB Patentanwälte · Rechtsanwälte
(86) International application number: PCT/JP2024/016854
(87) International publication number: WO 2025/027962

(57) **Abstract**

A medical device system (10) comprises an information device (14) and a medical device (12). The information device comprises a request signal transmission unit (68) that transmits a request signal to the medical device. The medical device comprises a request signal reception unit (44) that receives the request signal transmitted by the request signal transmission unit, and an information transmission unit (46) that transmits medical information held by the medical device in response to the reception of the request signal by the request signal reception unit. The information device further comprises an information acquisition unit (70) that can acquire the medical information transmitted by the information transmission unit.

## Description

### Technical Field

The present invention relates to a medical device system, a medical information acquisition method, a medical device, an information device, and a program.

### Background Art

JP 2020-160589 A, for example, discloses a medical device system that transmits information held by a measuring device (medical device) such as a sphygmomanometer to an information device such as a smartphone.

### Summary of Invention

A more excellent medical device system, medical information acquisition method, medical device, information device, and program are desired.

It is therefore an object of the present invention to solve the above-described problem.

(1) The first aspect of the present invention provides a medical device system including: an information device; and a medical device, wherein the information device includes a request signal transmission unit that transmits a request signal to the medical device, the medical device includes a request signal reception unit that receives the request signal transmitted by the request signal transmission unit, and an information transmission unit that transmits medical information held by the medical device in response to reception of the request signal by the request signal reception unit, and the information device further includes an information acquisition unit that is able to acquire the medical information transmitted by the information transmission unit.

According to the configuration described above, the medical information is transmitted from the medical device in response to the reception of the request signal transmitted from the information device, whereby the power consumption of the medical device can be reduced as compared with the case where the medical information is constantly transmitted from the medical device. That is, according to the configuration described above, a more excellent medical device system can be provided.

(2) In the medical device system according to (1), the request signal and the medical information may be wirelessly supplied.

(3) In the medical device system according to (2), the medical information may be supplied using a sound wave.

According to the configuration described above, the medical information can be supplied by transmitting and receiving sound waves, whereby the medical device and the information device can have a simple configuration.

(4) In the medical device system according to (2), the request signal may be supplied using an electromagnetic wave.

(5) In the medical device system according to (4), the electromagnetic wave may be light.

(6) In the medical device system according to (5), the request signal reception unit may receive the request signal when illuminance of the light is equal to or greater than a threshold value.

According to the configuration described above, the reception of a request signal based on unintended light such as an indoor light can be prevented.

(7) In the medical device system according to any one of (1) to (6), the medical device may further include a notification unit that provides a notification of notification information for notifying a user that the information transmission unit is transmitting the medical information.

According to the configuration described above, the user can know from the notification information that the medical information is being transmitted.

(8) In the medical device system according to (7), the notification unit may stop the notification of the notification information in response to completion of transmission of the medical information by the information transmission unit.

According to the configuration described above, the user can know from the stop of the notification information that the transmission of the medical information has been completed.

(9) In the medical device system according to any one of (1) to (8), the information transmission unit may continuously transmit the medical information for a predetermined time.

According to the configuration described above, the transmission of medical information can be easily controlled.

(10) In the medical device system according to any one of (1) to (9), the information transmission unit may further transmit identification information for identifying the medical device, the information device may further include an identification information determination unit that determines whether or not the identification information supplied from the medical device matches registration identification information registered in advance, and the information acquisition unit may acquire the medical information when the identification information determination unit determines that the identification information supplied from the medical device matches the registration identification information.

According to the configuration described above, even in a case where, for example, there is a plurality of medical devices, the information device can acquire only medical information of a specific medical device. In addition, this configuration can prevent the acquisition of another medical information by the information device.

(11) In the medical device system according to any one of (1) to (10), the information device may further include a storage unit that is able to store the medical information acquired by the information acquisition unit.

According to the configuration described above, the acquired medical information can be stored in the information device.

(12) In the medical device system according to any one of (1) to (11), the medical device may be formed so as to be attachable to and removable from a living body, and the information transmission unit may not transmit the medical information in a state where the medical device is attached to the living body, and may be able to transmit the medical information in response to removal of the medical device from the living body.

According to the configuration described above, in a case where the medical device acquires the medical information in a state where the medical device is attached to the living body, it is possible to prevent the medical information from being transmitted before or during the acquisition of the medical information by the medical device. Thus, it is possible to transmit the latest medical information after the medical device is removed from the living body.

(13) In the medical device system according to any one of (1) to (12), the medical device may be a liquid medicine administration device that administers a liquid medicine into a living body, the liquid medicine administration device may further include a liquid medicine administration determination unit that determines whether or not administration of the liquid medicine has been completed or has not been completed, and the information transmission unit may be able to transmit the medical information in response to determination by the liquid medicine administration determination unit that the administration of the liquid medicine has been completed or has not been completed.

According to the configuration described above, it is possible to transmit medical information regarding the administration of the liquid medicine by the liquid medicine administration device.

(14) In the medical device system according to any one of (1) to (13), the information device may further include a transmission unit that transmits the medical information acquired by the information acquisition unit to a server device via a network.

According to the configuration described above, the medical information can be stored in the server device.

(15) The second aspect of the present invention provides a medical information acquisition method including: a request signal transmission step of transmitting, by an information device, a request signal to a medical device; a request signal reception step of receiving, by the medical device, the request signal transmitted in the request signal transmission step; an information transmission step of transmitting, by the medical device, medical information held by the medical device in response to reception of the request signal in the request signal reception step; and an information acquisition step of acquiring, by the information device, the medical information transmitted in the information transmission step.

(16) The third aspect of the present invention provides a medical device including: a request signal reception unit that receives a request signal transmitted from an information device; and an information transmission unit that transmits medical information held by the medical device to the information device in response to reception of the request signal by the request signal reception unit.

(17) The fourth aspect of the present invention provides an information device including: a request signal transmission unit that transmits a request signal to a medical device holding medical information; and an information acquisition unit that acquires the medical information transmitted from the medical device in response to reception of the request signal by the medical device.

(18) The fifth aspect of the present invention provides a program for causing a computer provided in a medical device to execute: a request signal reception step of receiving a request signal transmitted from an information device; and an information transmission step of transmitting medical information held by the medical device to the information device in response to reception of the request signal in the request signal reception step.

(19) The sixth aspect of the present invention provides a program for causing a computer provided in an information device to execute: a request signal transmission step of transmitting a request signal to a medical device holding medical information; and an information acquisition step of acquiring the medical information transmitted from the medical device in response to reception of the request signal by the medical device.

According to the present invention, a more excellent medical device system, medical information acquisition method, medical device, information device, and program can be obtained.

### Brief Description of Drawings

Fig. 1 is a block diagram illustrating a medical device system according to an embodiment of the present invention.
Fig. 2 is an explanatory diagram of a configuration of a liquid medicine administration device.
Fig. 3 is a flowchart illustrating an example of a medical information acquisition method.

### Description of Embodiments

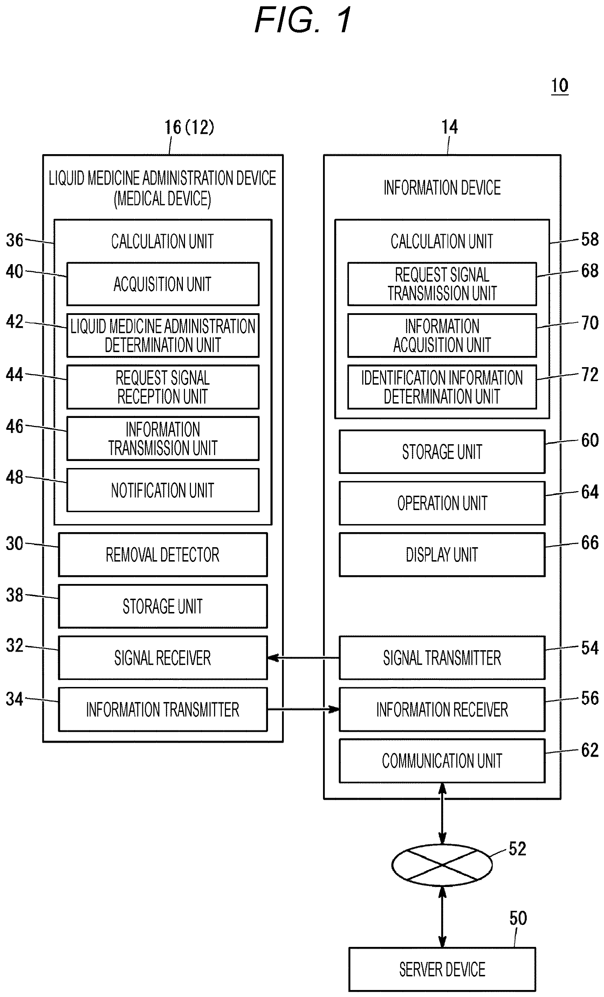

A medical device system, a medical information acquisition method, a medical device, an information device, and a program according to the present invention will be described with reference to Figs. 1 to 3. Fig. 1 is a block diagram illustrating a medical device system 10 according to an embodiment of the present invention.

As illustrated in Fig. 1, the medical device system 10 according to the present embodiment includes a medical device 12 and an information device 14. The medical device system 10 is a system for acquiring medical information (medical data) held by the medical device 12 by the information device 14. Specifically, the information device 14 wirelessly transmits a request signal to the medical device 12. The medical device 12 wirelessly transmits the medical information to the information device 14 in response to the reception of the request signal. The information device 14 acquires the medical information transmitted by the medical device 12.

The medical device system 10 may include one medical device 12 and one information device 14, or may include a plurality of medical devices 12 and a plurality of information devices 14. For example, in a case where the medical device system 10 is used in a hospital or the like, it can be assumed that the medical device system 10 includes a plurality of medical devices 12. In this case, for example, the medical information from the plurality of medical devices 12 can be individually managed by one information device 14. In addition, the medical device system 10 may include a plurality of types of medical devices 12 (for example, a liquid medicine administration device 16, a sphygmomanometer, a thermometer, and the like). In this case, for example, one information device 14 can manage medical information related to a plurality of types of medical devices 12 of a plurality of patients.

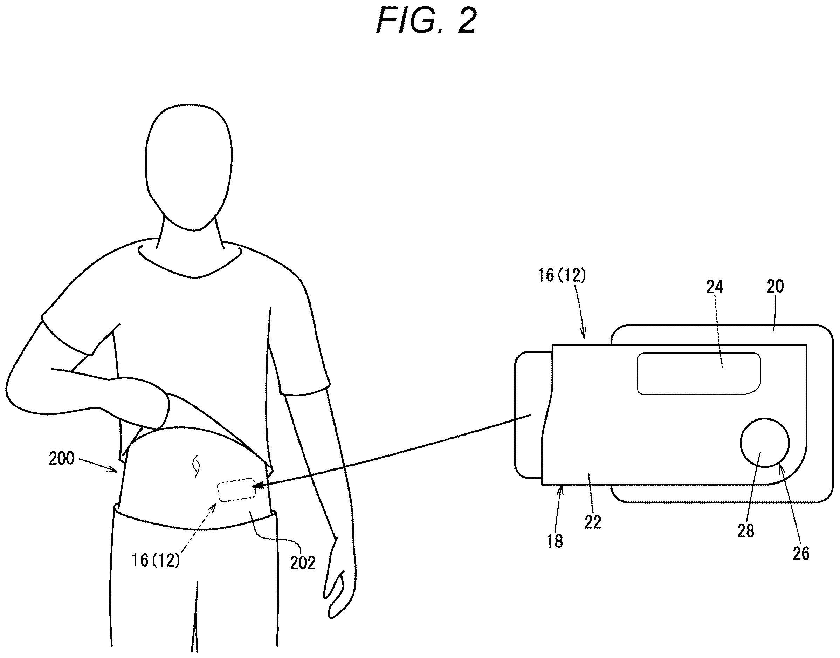

As illustrated in Fig. 2, the medical device 12 is a device attachable to and removable from a living body 200. In other words, the medical device 12 is used in a state of being in contact with the skin (body surface 202) of the living body 200. The medical device 12 can acquire medical information in a state of being attached to the living body 200. The present embodiment describes, as an example of the medical device 12, the liquid medicine administration device 16 for administering a liquid medicine to the living body 200. The medical device 12 is not limited to the liquid medicine administration device 16, and may be a sphygmomanometer, a thermometer, a blood glucose meter, a pulse oximeter, or the like. In this case, the medical information includes, for example, blood pressure, body temperature, blood glucose level, pulse rate, oxygen saturation, and the like.

The liquid medicine administration device 16 is a patch-type device to be affixed to the body surface 202. The liquid medicine administration device 16 continuously administers a liquid medicine into the living body 200 over a relatively long time (for example, about several minutes to several hours). The liquid medicine administration device 16 may intermittently administer the liquid medicine into the living body 200. Examples of the liquid medicine include protein preparations such as insulin preparations. Examples of a medical agent include a narcotic analgesic and a diuretic.

The liquid medicine administration device 16 includes a device body 18 and an attachment member 20. The device body 18 includes a housing 22, a prefilled syringe 24, and a puncture unit 26. The housing 22 accommodates various members including the prefilled syringe 24. The inside of the prefilled syringe 24 is filled with a liquid medicine in advance. The puncture unit 26 includes a puncture operation unit 28 for operating a puncture needle (not illustrated). When a user presses the puncture operation unit 28, the puncture needle protrudes from the housing 22. The attachment member 20 is fixed to the housing 22. The attachment member 20 has an adhesive surface that can be affixed to body surface 202.

In such a liquid medicine administration device 16, the user (for example, a patient) presses the puncture operation unit 28 in a state where the attachment member 20 is affixed to the body surface 202, whereby the living body 200 is punctured with the puncture needle. Thereafter, the administration of the liquid medicine from the prefilled syringe 24 to the living body 200 is started. When the administration of the liquid medicine is completed, the user removes the liquid medicine administration device 16 from the body surface 202.

As illustrated in Fig. 1, the liquid medicine administration device 16 further includes a removal detector 30, a signal receiver 32, an information transmitter 34, a calculation unit 36, and a storage unit 38. Note that the liquid medicine administration device 16 may also include components other than the above-mentioned components, but the description thereof is omitted here. In the storage unit 38, a program for causing a computer to execute the medical information acquisition method according to the present embodiment can be installed.

The removal detector 30 detects that the liquid medicine administration device 16 has been removed from the living body 200. The removal detector 30 may include, for example, at least one of a temperature sensor, an illuminance sensor, a pressure sensor, a capacitance sensor, and a mechanical switch.

The temperature sensor can detect a temperature change before and after the liquid medicine administration device 16 is removed from the living body 200. The illuminance sensor can detect a change in illuminance (change in light amount) before and after the liquid medicine administration device 16 is removed from the living body 200.

A light receiving element of the illuminance sensor can be disposed so as to be oriented in, for example, a direction different from the body surface 202 in a state where the liquid medicine administration device 16 is attached to the living body 200 (attached state of the liquid medicine administration device 16). In this case, the light receiving element of the illuminance sensor is covered with clothing in the attached state of the liquid medicine administration device 16. On the other hand, the light receiving element of the illuminance sensor is exposed from the clothing in a state where the liquid medicine administration device 16 is removed from the living body 200 (removal state of the liquid medicine administration device 16). Therefore, the illuminance measured by the illuminance sensor in the removal state of the liquid medicine administration device 16 is larger than the illuminance measured by the illuminance sensor in the attached state of the liquid medicine administration device 16.

Note that the light receiving element of the illuminance sensor may be disposed so as to face the body surface 202 in the attached state of the liquid medicine administration device 16. In this case, the light receiving element of the illuminance sensor is covered with the body surface 202 in the attached state of the liquid medicine administration device 16. On the other hand, the light receiving element of the illuminance sensor is exposed to the outside in the removal state of the liquid medicine administration device 16. Therefore, the illuminance measured by the illuminance sensor in the removal state of the liquid medicine administration device 16 is larger than the illuminance measured by the illuminance sensor in the attached state of the liquid medicine administration device 16.

The pressure sensor can detect a change in pressure acting on the device body 18 or the attachment member 20 before and after the liquid medicine administration device 16 is removed from the body surface 202. The capacitance sensor can detect a change in capacitance before and after the liquid medicine administration device 16 is removed from the body surface 202. The mechanical switch includes, for example, a mechanism that operates when the liquid medicine administration device 16 is removed from the living body 200, and can detect the removal of the liquid medicine administration device 16 from the living body 200 by detecting the operation of the mechanism.

The signal receiver 32 can receive a request signal transmitted from the information device 14. The information transmitter 34 can transmit predetermined information (identification information and medical information).

The calculation unit 36 includes a processor such as a central processing unit (CPU) or a graphics processing unit (GPU). That is, the calculation unit 36 includes processing circuitry. The calculation unit 36 includes an acquisition unit 40, a liquid medicine administration determination unit 42, a request signal reception unit 44, an information transmission unit 46, and a notification unit 48. The acquisition unit 40, the liquid medicine administration determination unit 42, the request signal reception unit 44, the information transmission unit 46, and the notification unit 48 can be implemented by executing a program stored in the storage unit 38 by the calculation unit 36.

Note that at least a part of the acquisition unit 40, the liquid medicine administration determination unit 42, the request signal reception unit 44, the information transmission unit 46, and the notification unit 48 may be implemented by an integrated circuit such as an application specific integrated circuit (ASIC) or a field-programmable gate array (FPGA). At least a part of the acquisition unit 40, the liquid medicine administration determination unit 42, the request signal reception unit 44, the information transmission unit 46, and the notification unit 48 may be composed of an electronic circuit including a discrete device.

The storage unit 38 can include a volatile memory (not illustrated) and a non-volatile memory (not illustrated). Examples of the volatile memory include a random access memory (RAM). The volatile memory is used as a working memory of the processor, and temporarily stores data and the like necessary for processing or calculation. Examples of the non-volatile memory include a read only memory (ROM) and a flash memory. The non-volatile memory is used as a memory for storage, and stores a program, a table, a map, and the like. At least a part of the storage unit 38 may be provided in the processor, the integrated circuit, or the like as described above.

In the storage unit 38, identification information of the liquid medicine administration device 16 is stored in advance. The identification information is, for example, a unique serial number or the like given to the liquid medicine administration device 16.

The acquisition unit 40 can acquire information (medical information) regarding administration of the liquid medicine by the liquid medicine administration device 16. The medical information can include, for example, information such as the date and time when the liquid medicine was administered, the type of the liquid medicine, the dose of the liquid medicine, and the occurrence of an error during the administration of the liquid medicine. The medical information acquired by the acquisition unit 40 is stored in the storage unit 38. The liquid medicine administration determination unit 42 determines whether the administration of the liquid medicine has been completed or has not been completed. Here, the situation in which the administration of the liquid medicine has not been completed means the occurrence of an abnormality in the administration of the liquid medicine. Examples of the abnormality in the administration of the liquid medicine include an abnormality in which the liquid medicine is not normally administered due to blockage of the puncture needle.

The request signal reception unit 44 receives a request signal on the basis of the signal received by the signal receiver 32. The information transmission unit 46 transmits predetermined information (identification information and medical information) via the information transmitter 34 in response to the reception by the request signal reception unit 44. The information transmission unit 46 can transmit predetermined information on the basis of the determination by the liquid medicine administration determination unit 42 that the administration of the liquid medicine has been completed or has not been completed. That is, when the liquid medicine administration determination unit 42 determines that the administration of the liquid medicine has been completed or has not been completed, the transmission of information indicating that the administration of the liquid medicine has been completed or has not been completed from the information transmission unit 46 is allowed. The information transmission unit 46 can continuously transmit information for a predetermined time, for example. Note that the information transmission unit 46 can also transmit information other than the identification information and the medical information.

When the liquid medicine administration determination unit 42 determines that the administration of the liquid medicine has been completed or has not been completed, the notification unit 48 provides liquid medicine administration information for notifying the user that the administration of the liquid medicine has been completed or has not been completed. In addition, the notification unit 48 provides notification information for notifying the user that the information transmission unit 46 is transmitting the medical information. The notification unit 48 emits a sound from, for example, a speaker or a buzzer (not illustrated). The notification unit 48 may, for example, turn on or blink an indicator lamp (not illustrated) provided in the liquid medicine administration device 16. The notification unit 48 may, for example, display predetermined characters, symbols, graphics, and the like on a display unit (not illustrated) provided in the liquid medicine administration device 16. The notification unit 48 may, for example, vibrate the liquid medicine administration device 16. In addition, the notification unit 48 may provide a notification in combination of the above two or more notification methods. Furthermore, the mode of notification may be distinguished between completion and incompletion (abnormality) of the administration of the liquid medicine. That is, for example, the notification unit may change the pitch of the sound, the color of the indicator lamp, the blinking speed of the indicator lamp, the vibration rhythm, and the like to distinguish the mode of notification between completion and incompletion (abnormality) of the administration of the liquid medicine. As a result, the user can recognize whether or not the liquid medicine has been normally administered.

The notification unit 48 stops the notification of the notification information in response to the completion of the transmission of the information by the information transmission unit 46. For example, the notification unit 48 can start the notification of the notification information at the same time as the start of transmission of the medical information by the information transmission unit 46 and stop the notification of the notification information at the same time as the stop of the transmission of the information by the information transmission unit 46. Note that the notification unit 48 may stop the notification of the notification information after a predetermined time has elapsed since the transmission of the information by the information transmission unit 46 was stopped. The notification time by the notification unit 48 may be longer or shorter than the information transmission time by the information transmission unit 46.

Examples of the information device 14 include, but are not limited to, a smartphone and a tablet. The information device 14 can acquire medical information from the liquid medicine administration device 16. The information device 14 can manage the medical information acquired from the liquid medicine administration device 16.

The information device 14 can communicate with a server device 50 via a network 52 such as the Internet. The information device 14 can transmit the medical information to the server device 50. The server device 50 can manage the medical information. The server device 50 can communicate with a terminal device (computer) (not illustrated) provided in a hospital or the like via the network 52, for example. A medical worker in a hospital or the like can confirm medical information of a user (patient) stored in the server device 50 using the terminal device.

The information device 14 includes a signal transmitter 54, an information receiver 56, a calculation unit 58, a storage unit 60, a communication unit 62 (transmission unit), an operation unit 64, and a display unit 66. The information device 14 may include components other than these components, but the description thereof is omitted here. In the information device 14, a program for causing a computer to execute the medical information acquisition method according to the present embodiment can be installed in advance. More specifically, a medical information management application program (medical information management app) to be described later can be installed in the information device 14 in advance.

The signal transmitter 54 can transmit a request signal to the liquid medicine administration device 16. The information receiver 56 can receive predetermined information (identification information and medical information) transmitted from the liquid medicine administration device 16.

The calculation unit 58 is composed of a processor such as a CPU or a GPU. That is, the calculation unit 58 is composed of a processing circuit. The calculation unit 58 includes a request signal transmission unit 68, an information acquisition unit 70, and an identification information determination unit 72. The request signal transmission unit 68, the information acquisition unit 70, and the identification information determination unit 72 can be implemented by executing a program stored in the storage unit 60 by the calculation unit 58.

Note that at least a part of the request signal transmission unit 68, the information acquisition unit 70, and the identification information determination unit 72 may be implemented by an integrated circuit such as an ASIC or an FPGA. Furthermore, at least a part of the request signal transmission unit 68, the information acquisition unit 70, and the identification information determination unit 72 may be composed of an electronic circuit including a discrete device.

The storage unit 60 includes a volatile memory (not illustrated) and a non-volatile memory (not illustrated). Examples of the volatile memory include a RAM. The volatile memory is used as a working memory of the processor, and temporarily stores data and the like necessary for processing or calculation. Examples of the non-volatile memory include a ROM and a flash memory. The non-volatile memory is used as a memory for storage, and stores a program, a table, a map, and the like. At least a part of the storage unit 60 may be provided in the processor, the integrated circuit, or the like as described above.

The communication unit 62 includes, for example, a communication module (not illustrated). The communication unit 62 can transmit and receive data via the network 52 such as the Internet.

The operation unit 64 is used when the user operates the information device 14. The display unit 66 includes a display element (not illustrated). As the display element, for example, a liquid crystal display element, an organic electroluminescence display element, or the like is used. The operation unit 64 and the display unit 66 can be composed of a touch panel (not illustrated) provided with such a display element, but is not limited thereto. The operation unit 64 may be composed of a keyboard, a mouse, or the like.

The request signal transmission unit 68 can transmit a request signal to the liquid medicine administration device 16 via the signal transmitter 54. The information acquisition unit 70 can acquire predetermined information (identification information and medical information) transmitted from the liquid medicine administration device 16 via the information receiver 56. The identification information determination unit 72 determines whether or not the identification information acquired by the information acquisition unit 70 matches registration identification information registered in advance.

The registration identification information is identification information for identifying the liquid medicine administration device 16 registered in a database of the storage unit 60. The identification information for identifying the liquid medicine administration device 16 can be registered in the database by an input performed by the user using the operation unit 64, for example. Furthermore, the identification information for identifying the liquid medicine administration device 16 can be registered in the database by, for example, reading a barcode or a QR code (registered trademark) including the identification information with a camera (not illustrated) provided in the information device 14. Furthermore, the identification information for identifying the liquid medicine administration device 16 may be stored in advance in the server device 50, and may be registered in the database by being acquired from the server device 50 via the network 52.

The request signal is supplied using, for example, radio waves used in near field communication (NFC). In this case, the signal transmitter 54 has an NFC antenna for transmission, and the signal receiver 32 has an NFC antenna for reception. The frequency of the radio wave used in NFC is, for example, 13.56 MHz.

The identification information and the medical information are supplied using, for example, sound waves. The sound wave may be an ultrasound wave or a sound wave in an audible frequency band. In this case, the information transmitter 34 includes a speaker, and the information receiver 56 includes a microphone. When a sound wave in an audible frequency band is used, the sound wave is also used to provide notification information.

The request signal may be supplied using sound waves. The identification information and the medical information may be supplied using radio waves used in NFC. Alternatively, the request signal, the identification information, and the medical information may be supplied using electromagnetic waves other than radio waves used in NFC. In this case, the request signal, the identification information, and the medical information can be supplied by Bluetooth (registered trademark), infrared communication, wireless LAN, or the like.

As described above, a program for causing a computer included in the information device 14 to execute the medical information acquisition method according to the present embodiment can be installed in advance in the information device 14. More specifically, a medical information management application program (medical information management app) can be installed in the information device 14 in advance. The medical information management app can be downloaded via a website or the like, for example, but is not limited thereto.

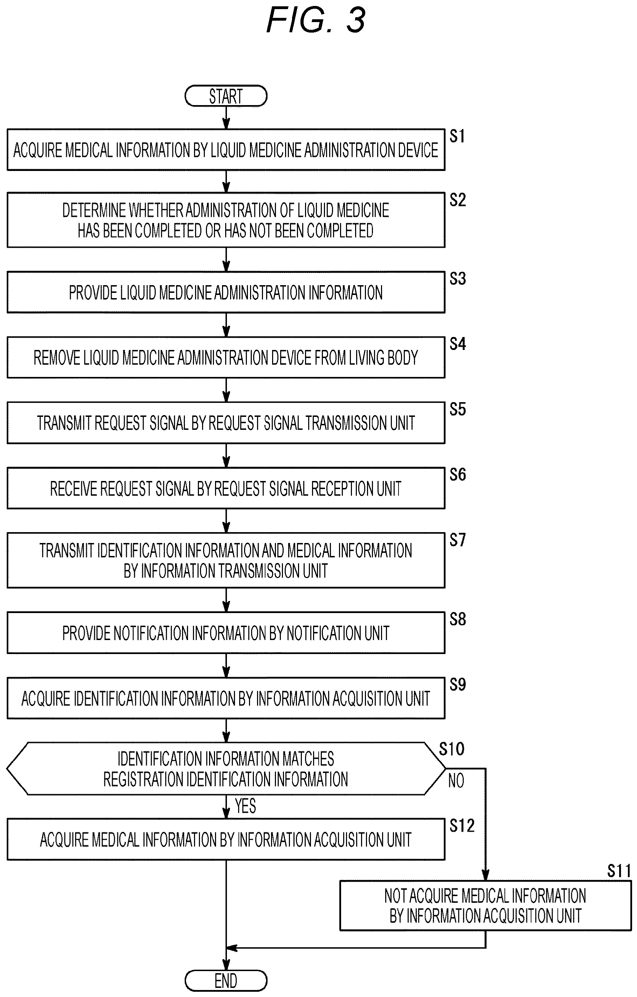

Next, the medical information acquisition method according to the present embodiment will be described. Fig. 3 is a flowchart illustrating an example of the medical information acquisition method.

In step S1, the liquid medicine administration device 16 acquires medical information. Specifically, the user wears the liquid medicine administration device 16 on the living body 200, and the liquid medicine is administered by the liquid medicine administration device 16. The acquisition unit 40 of the liquid medicine administration device 16 acquires medical information such as a liquid medicine dose. During the administration of the liquid medicine, the transmission of medical information from the information transmitter 34 by the information transmission unit 46 is restricted. Thereafter, the processing proceeds to step S2.

In step S2, the liquid medicine administration determination unit 42 determines whether the administration of the liquid medicine has been completed or has not been completed. Thereafter, the processing proceeds to step S3.

In step S3, the notification unit 48 provides a notification about the liquid medicine administration information. As a result, the user can know that the administration of the liquid medicine has been completed or has not been completed (is abnormal). Thereafter, the processing proceeds to step S4.

In step S4, the liquid medicine administration device 16 is removed from the living body 200. In this case, the removal detector 30 detects that the liquid medicine administration device 16 has been removed from the living body 200. The information transmission unit 46 enables the transmission of medical information on the basis of the detection by the removal detector 30 that the liquid medicine administration device 16 has been removed from the living body 200. Thereafter, the processing proceeds to step S5.

In step S5 (request signal transmission step), the information device 14 transmits a request signal to the liquid medicine administration device 16. Specifically, the user performs a medical information acquisition process using the medical information management app of the information device 14. When the medical information acquisition process is performed by the medical information management app, the request signal transmission unit 68 transmits a request signal via the signal transmitter 54. The user brings the information device 14 close to the liquid medicine administration device 16 as necessary. When the request signal is supplied using radio waves used in NFC, the information device 14 is preferably brought close to the liquid medicine administration device 16, and information for prompting such an operation may be displayed on the display unit 66 of the information device 14. In step S5, in a case where there is a plurality of pieces of registration identification information, the registration identification information of the liquid medicine administration device 16 from which medical information is scheduled to be acquired this time may be selected in the medical information management app. Thereafter, the processing proceeds to step S6.

In step S6 (request signal reception step), the signal receiver 32 receives the request signal, so that the request signal reception unit 44 receives the request signal. Thereafter, the processing proceeds to step S7.

In step S7 (information transmission step), the information transmission unit 46 transmits the identification information and the medical information of the liquid medicine administration device 16 via the information transmitter 34 in response to the reception of the request signal. Thus, the information receiver 56 receives the identification information and the medical information. In step S8, the notification unit 48 provides notification information. As a result, the user can know that the identification information and the medical information are transmitted from the liquid medicine administration device 16. Thereafter, the processing proceeds to step S9.

In step S9, the information acquisition unit 70 acquires the identification information received by the information receiver 56. Thereafter, the processing proceeds to step S10.

In step S10, the identification information determination unit 72 determines whether or not the acquired identification information matches the registration identification information. When the registration identification information is selected in the medical information management app in step S5, the identification information determination unit 72 determines whether or not the acquired identification information matches the selected registration identification information. When a plurality of pieces of registration identification information is stored in the storage unit 60 and the registration identification information is not selected, the identification information determination unit 72 determines whether or not the acquired identification information matches one of the plurality of pieces of registration identification information. When the identification information determination unit 72 determines that the identification information does not match the registration identification information (NO in step S10), the processing proceeds to step S11.

In step S11, the information acquisition unit 70 does not acquire the medical information received via the information receiver 56. Thus, it is possible to prevent erroneous acquisition of medical information held by another medical device other than the liquid medicine administration device 16. In this case, the information device 14 may notify the user of error information in order to let the user know that the medical information has failed to be acquired. Thereafter, the processing illustrated in Fig. 3 ends.

When the identification information determination unit 72 determines that the identification information matches the registration identification information (YES in step S10), the processing proceeds to step S12.

In step S12 (information acquisition step), the information acquisition unit 70 acquires the medical information received via the information receiver 56. The acquired medical information is stored in the storage unit 60. The medical information may be transmitted from the communication unit 62 to the server device 50 via the network 52. Thereafter, the processing illustrated in Fig. 3 ends.

According to the present embodiment, the medical information is transmitted from the liquid medicine administration device 16 in response to the reception of the request signal transmitted from the information device 14, whereby it is possible to suppress the power consumption of the liquid medicine administration device 16 as compared with a case where the medical information is constantly transmitted from the liquid medicine administration device 16. That is, according to the present embodiment, an excellent medical device system 10 can be provided.

The medical information may be supplied using sound waves. According to the configuration described above, the medical information can be supplied by transmission and reception of sound waves, whereby the liquid medicine administration device 16 and the information device 14 can have a simple configuration.

The liquid medicine administration device 16 includes a notification unit 48 that provides notification information for notifying the user that the information transmission unit 46 is transmitting the medical information. According to the configuration described above, the user can know from the notification information that the medical information is being transmitted.

The notification unit 48 stops the notification of the notification information in response to the completion of the transmission of the medical information by the information transmission unit 46. According to the configuration described above, the user can know from the stop of the notification information that the transmission of the medical information has been completed.

The information transmission unit 46 can continuously transmit medical information for a predetermined time. According to the configuration described above, the transmission of medical information can be easily controlled.

The information transmission unit 46 further transmits identification information for identifying the liquid medicine administration device 16, and the information device 14 further includes an identification information determination unit 72 that determines whether or not the identification information supplied from the liquid medicine administration device 16 matches registration identification information registered in advance. The information acquisition unit 70 acquires medical information when the identification information determination unit 72 determines that the identification information supplied from the liquid medicine administration device 16 matches the registration identification information. According to the configuration described above, even in a case where, for example, there is a plurality of medical devices, the information device 14 can acquire only the medical information of the specific liquid medicine administration device 16. In addition, this configuration can prevent the acquisition of medical information from another medical device by the information device 14.

The information device 14 includes a storage unit 60 that can store the medical information acquired by the information acquisition unit 70. According to the configuration described above, the acquired medical information can be stored in the information device 14.

The liquid medicine administration device 16 is formed so as to be attachable to and removable from the living body 200, and the information transmission unit 46 does not transmit medical information in a state where the liquid medicine administration device 16 is attached to the living body 200, and can transmit medical information on the basis of the removal of the liquid medicine administration device 16 from the living body 200. According to the configuration described above, it is possible to prevent the medical information from being transmitted before or during the acquisition of the medical information by the liquid medicine administration device 16. Thus, it is possible to transmit the latest medical information after the liquid medicine administration device 16 is removed from the living body 200.

The liquid medicine administration device 16 further includes a liquid medicine administration determination unit 42 that determines whether the administration of the liquid medicine has been completed or has not been completed. The information transmission unit 46 can transmit the medical information on the basis of the determination by the liquid medicine administration determination unit 42 that the administration of the liquid medicine has been completed or has not been completed. According to the configuration described above, it is possible to transmit medical information regarding the administration of the liquid medicine by the liquid medicine administration device 16.

The information device 14 further includes a communication unit 62 that transmits the medical information acquired by the information acquisition unit 70 to the server device 50 via the network 52. According to the configuration described above, the medical information can be stored in the server device 50.

### (Modification)

When the request signal is supplied using electromagnetic waves, the electromagnetic waves may be light. In this case, the signal transmitter 54 includes a light emitting unit, and the signal receiver 32 includes a light receiving unit. Further, the request signal reception unit 44 receives the request signal when the illuminance of the light received by the light receiving unit is equal to or greater than a predetermined threshold value. According to the configuration described above, the reception of a request signal based on unintended light such as an indoor light can be prevented. Examples of the light used to supply the request signal include, but are not limited to, infrared light, visible light, and the like.

In such a modification, the request signal reception unit 44 may receive the request signal on the basis of a wavelength or the like of light received by the light receiving unit. That is, when light having a specific wavelength is received by the light receiving unit, the request signal may be received.

Note that the present invention is not limited to the above disclosure, and various configurations can be adopted without departing from the gist of the present invention.

## Claims

1. A medical device system comprising: an information device; and a medical device, wherein
the information device comprises a request signal transmission unit that transmits a request signal to the medical device,
the medical device comprises
a request signal reception unit that receives the request signal transmitted by the request signal transmission unit, and
an information transmission unit that transmits medical information held by the medical device in response to reception of the request signal by the request signal reception unit, and
the information device further comprises an information acquisition unit that is able to acquire the medical information transmitted by the information transmission unit.

2. The medical device system according to claim 1, wherein
the request signal and the medical information are wirelessly supplied.

3. The medical device system according to claim 2, wherein
the medical information is supplied using a sound wave.

4. The medical device system according to claim 2, wherein
the request signal is supplied using an electromagnetic wave.

5. The medical device system according to claim 4, wherein
the electromagnetic wave is light.

6. The medical device system according to claim 5, wherein
the request signal reception unit receives the request signal when illuminance of the light is equal to or greater than a threshold value.

7. The medical device system according to claim 1, wherein
the medical device further comprises a notification unit that provides a notification of notification information for notifying a user that the information transmission unit is transmitting the medical information.

8. The medical device system according to claim 7, wherein
the notification unit stops the notification of the notification information in response to completion of transmission of the medical information by the information transmission unit.

9. The medical device system according to claim 1, wherein
the information transmission unit continuously transmits the medical information for a predetermined time.

10. The medical device system according to claim 1, wherein
the information transmission unit further transmits identification information for identifying the medical device,
the information device further comprises an identification information determination unit that determines whether or not the identification information supplied from the medical device matches registration identification information registered in advance, and
the information acquisition unit acquires the medical information when the identification information determination unit determines that the identification information supplied from the medical device matches the registration identification information.

11. The medical device system according to claim 1, wherein
the information device further comprises a storage unit that is able to store the medical information acquired by the information acquisition unit.

12. The medical device system according to claim 1, wherein
the medical device is formed so as to be attachable to and removable from a living body, and
the information transmission unit does not transmit the medical information in a state where the medical device is attached to the living body, and is able to transmit the medical information in response to removal of the medical device from the living body.

13. The medical device system according to claim 1, wherein
the medical device is a liquid medicine administration device that administers a liquid medicine into a living body,
the liquid medicine administration device further comprises a liquid medicine administration determination unit that determines whether or not administration of the liquid medicine has been completed or has not been completed, and
the information transmission unit is able to transmit the medical information in response to determination by the liquid medicine administration determination unit that the administration of the liquid medicine has been completed or has not been completed.

14. The medical device system according to any one of claims 1 to 13, wherein
the information device further comprises a transmission unit that transmits the medical information acquired by the information acquisition unit to a server device via a network.

15. A medical information acquisition method comprising:
a request signal transmission step of transmitting, by an information device, a request signal to a medical device;
a request signal reception step of receiving, by the medical device, the request signal transmitted in the request signal transmission step;
an information transmission step of transmitting, by the medical device, medical information held by the medical device in response to reception of the request signal in the request signal reception step; and
an information acquisition step of acquiring, by the information device, the medical information transmitted in the information transmission step.

16. A medical device comprising:
a request signal reception unit that receives a request signal transmitted from an information device; and an information transmission unit that transmits medical information held by the medical device to the information device in response to reception of the request signal by the request signal reception unit.

17. An information device comprising:
a request signal transmission unit that transmits a request signal to a medical device holding medical information; and
an information acquisition unit that acquires the medical information transmitted from the medical device in response to reception of the request signal by the medical device.

18. A program for causing a computer provided in a medical device to execute:
a request signal reception step of receiving a request signal transmitted from an information device; and
an information transmission step of transmitting medical information held by the medical device to the information device in response to reception of the request signal in the request signal reception step.

19. A program for causing a computer provided in an information device to execute:
a request signal transmission step of transmitting a request signal to a medical device holding medical information; and
an information acquisition step of acquiring the medical information transmitted from the medical device in response to reception of the request signal by the medical device.
